(19) Europäisches Patentamt
European Patent Office
Office européen des brevets

(11) Veröffentlichungsnummer : **0 192 598**
**B1**

(12) # EUROPÄISCHE PATENTSCHRIFT

(45) Veröffentlichungstag der Patentschrift : **13.12.89**

(51) Int. Cl.[4] : **C 07 J 41/00**, A 61 K 31/565

(21) Anmeldenummer : **86730025.3**

(22) Anmeldetag : **20.02.86**

(54) **11 Beta-N,N-Dimethylaminophenyl-Estradiene, deren Herstellung und diese enthaltende pharmazeutische Präparate.**

(30) Priorität : **22.02.85 DE 3506785**

(43) Veröffentlichungstag der Anmeldung : **27.08.86 Patentblatt 86/35**

(45) Bekanntmachung des Hinweises auf die Patenterteilung : **13.12.89 Patentblatt 89/50**

(84) Benannte Vertragsstaaten : **AT BE CH DE FR GB IT LI LU NL SE**

(56) Entgegenhaltungen :
**EP-A- 0 116 974**
**EP-A- 0 147 361**
**US-A- 4 386 085**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Ottow, Eckhard, Dr.**
**Sonnenallee 124**
**D-1000 Berlin 44 (DE)**
Erfinder : **Neef, Günter, Dr.**
**Darmstädter Strasse 9**
**D-1000 Berlin 15 (DE)**
Erfinder : **Rohde, Ralph, Dr.**
**Schwatlostrasse 14**
**D-1000 Berlin 45 (DE)**
Erfinder : **Wiechert, Rudolf, Prof.**
**Petzower Strasse 8A**
**D-1000 Berlin 39 (DE)**
Erfinder : **Beier, Sybille, Dr.**
**Uhlandstrasse 121**
**D-1000 Berlin 31 (DE)**
Erfinder : **Elger, Walter, Dr.**
**Schorlemmer Allee**
**D-1000 Berlin 33 (DE)**
Erfinder : **Henderson, David, Dr.**
**Jahnstrasse 17**
**D-1000 Berlin 28 (DE)**

**EP 0 192 598 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 11β-Phenyl-Estradiene der allgemeinen Formel (I),

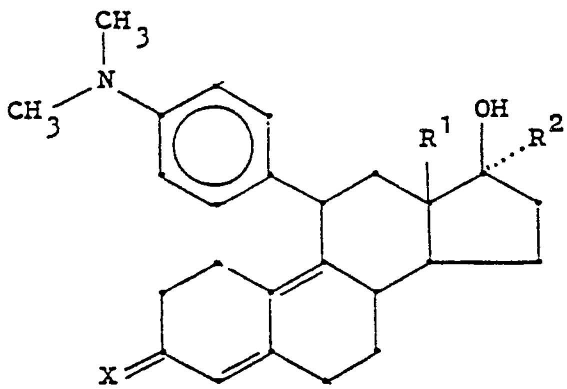

(I)

worin

X ein Sauerstoffatom oder eine Hydroxyiminogruppe N~OH, wobei die Hydroxygruppe syn- oder antiständig sein kann,

$R^1$ eine Methyl- oder Ethylgruppe,

$R^2$ eine $-C{\equiv}C-CH{=}CH_2$, eine $-CH_2-(CH_2)_n-Y-R^3$ oder eine $-CH{=}CH-(CH_2)_m-Y-R^3$-Gruppe bedeuten, wobei Y für ein Sauerstoff- oder Schwefelatom, n für die Ziffern 0 oder 1, m für die Ziffern 1, 2 oder 3 und $R^3$ für ein Wasserstoffatom, für einen Alkyl- oder Acylrest mit jeweils 1 bis 4 Kohlenstoffatomen steht, mit der Maßgabe, daß wenn m für 1 und Y für ein Sauerstoffatom stehen, $R^3$ ein Alkylrest mit 1 bis 4 Kohlenstoffatomen bedeutet, Verfahren zu deren Herstellung und diese Verbindungen enthaltende pharmazeutische Präparate gemäß den Patentansprüchen.

11β-Phenyl-Estradiene sind bereits bekannt. So werden beispielsweise 11β-Aryl-17α-propinyl- und -ethinyl-4,9(10)-estradiene in der europäischen Patentanmeldung 82400025.1 (Publikations-Nr. 0 057 115) und der US-Patentschrift 4 386 085 sowie 11β-Phenyl-17α-(3-hydroxyproyl)-4,9(10)-estradiene in der europäischen Patentanmeldung 84101721.3 (Publikations-Nr. 0 116 974) beschrieben. Diese Verbindungen besitzen eine starke Affinität zum Gestagenrezeptor, ohne selbst gestagene Aktivität zu besitzen. Sie sind kompetitive Antagonisten des Progesterons (Anti-Gestagene) und sind zur Auslösung von Aborten geeignet, da sie das zur Aufrechterhaltung der Schwangerschaft erforderliche Progesteron vom Rezeptor verdrängen. Sie sind deshalb wertvoll und interessant im Hinblick auf ihre Verwendung zur postcoitalen Fertilitätskontrolle.

Sie können auch gegen hormonelle Unregelmäßigkeiten, zur Menstruationsauslösung und zur Geburtseinleitung eingesetzt werden.

Die in der europäischen Patentanmeldung 84101721.3 aufgeführten Verbindungen besitzen zusätzlich zu ihren antigestagenen Eigenschaften noch antimineralcorticoide Wirkungen.

Die zuerst genannten 11β-Aryl-17α-propinyl- und -ethinyl-4,9(10)-estradiene weisen dagegen eine antiglucocorticoide Aktivität auf und können somit auch als Arzneimittel zur Therapie corticoid-induzierter Störungen (Glaukom) sowie zur Bekämpfung von Nebenwirkungen, die bei langfristiger Behandlung mit Glucocorticoiden auftreten (Cushing-Syndrom), eingesetzt werden. Sie ermöglichen daher auch die auf eine Supersekretion der Glucocorticoide zurückzuführenden Störungen, vor allem die Adipositas, Arteriosklerose, Osteoporose, Diabetes sowie die Insomnie zu bekämpfen.

Es wurde nun gefunden, daß die neuen Verbindungen der allgemeinen Formel I überraschenderweise deutlich stärkere Wirkungen als die bisher bekannten Verbindungen dieser Stoffklasse zeigen. Dabei kann einerseits sowohl die antigestagene als auch die antiglucocorticoide Wirkung stärker sein, andererseits können aber auch beide Wirkungen im Vergleich zu denen der bisher bekannten Verbindungen verstärkt sein.

Zur Kennzeichnung der antigestagenen Wirkung wurde die abortive Wirkung bestimmt.

Die Versuche wurden an weiblichen Ratten im Gewicht von ca. 200 g durchgeführt. Nach erfolgter Anpaarung wurde der Schwangerschaftsbeginn durch Nachweis von Spermien in Vaginalabstrichen gesichert. Der Tag des Spermiennachweises gilt als Tag 1 der Gravidität (= d1 p. c.).

Die Behandlung der Tiere mit der jeweils zu testenden Substanz bzw. dem Lösungsmittel erfolgte nach der Nidation der Blastocysten von d5 p. c. bis d7 p. c. An d9 p. c. wurden die Tiere getötet und die Uteri auf Implantate und Resorptionsstellen hin untersucht. Von allen Uteri wurden Fotos angefertigt. Das Fehlen von Implantaten wurde als Abort gewertet.

Die Testsubstanzen wurden in einem Benzylbenzoat-Rizinusöl-Gemisch (Verhältnis 1 + 9) gelöst. Das Vehikelvolumen pro Einzeldosis betrug 0,2 ml. Die Behandlung erfolgte subcutan (s. c.).

Die Überlegenheit der erfindungsgemäßen Verbindungen soll durch Vergleich der biologischen

Eigenschaften von der erfindungsgemäßen Verbindung 11β-(4-Dimethylaminophenyl)-17α-methoxymethyl-17β-hydroxy-4,9-estradien-3-on (A), dem in EP 82400025.1 beschriebenen 11β-(4-Dimethylaminophenyl)-17β-hydroxy-17α(propin-1-yl)-4,9(10)-estradien-3-on RU 38486 (B) und dem in EP 84101721.3 beschriebenen 11β-(4-Dimethylaminophenyl)-17β-hydroxy-17α-(3-hydroxypropyl)-4,9(10)-estradien-3-on (C) gezeigt werden :

Tabelle 1

Abortivtest bei der graviden Ratte

| Substanz | Dosis mg/Tier/Tag s.c. | Abortrate n-Abort-positiv / n Gesamt |
|---|---|---|
| A | 10,0 | 4 / 4 |
| | 3,0 | 3 / 3 |
| | 1,0 | 3 / 3 |
| B | 10,0 | 4 / 4 |
| | 3,0 | 4 / 4 |
| | 1,0 | 2 / 4 |
| C | 10,0 | 4 / 4 |
| | 3,0 | 4 / 4 |
| | 1,0 | 0 / 4 |

Aus Tabelle 1 ist zu entnehmen, daß nur die erfindungsgemäße Verbindung A bei einer Dosis von 1 mg abortiv voll wirksam ist, d. h. sie ist um den Faktor 3 wirksamer als die Verbindungen des Standes der Technik (Endocrinology 1984, 239).

Zur Kennzeichnung der antiglucocorticoiden Wirkung wurde der Einfluß der erfindungsgemäßen Substanzen auf die Tyrosin-Aminotransferase bestimmt. Das Test-System basiert auf einer Messung der Aktivität des Leberenzyms Tyrosin Aminotransferase (TAT) in Kulturen von RHC (Rat Hepatoma Cells) Zellen. Das Enzym katalysiert den ersten Schritt in der Verstoffwechselung von Tyrosin und ist sowohl in der Leber als auch in Hepatomzellen durch Glucocorticoide induzierbar. Die Aktivität ist in Rohextrakten leicht meßbar (Granner und Tomkins, (1970) Meth. Enzymol. 15, 633). Das Enzym überführt die Aminogruppe von Tyrosin auf 2-Oxoglutarsäure. Dabei entstehen Glutaminsäure und p-Hydroxyphenylpyruvat. In alkalischer Lösung wird aus p-Hydroxyphenylpyruvat das stabilere p-Hydroxybenzaldehyd gebildet, dessen Absorption bei 331 nm gemessen wird. Die TAT-Aktivität in RHC-Zellen zeigt eine dosisabhängige Induktion mit Cortisol (max. Akt. bei $10^{-6}$ M) oder Dexamethason (max. Akt. bei $10^{-7}$ M). Die Aktivität läßt sich um den Faktor 4-6 über den Basalwert stimulieren. Gleichzeitige Behandlung mit Corticoid und Antiglucocorticoid führt zu einer Abnahme der TAT-Aktivität.

Die erfindungsgemäße Verbindung A zeigt in diesem Test die gleiche Aktivität wie RU 38.486 (B), einer Substanz, die als Standard anzusehen ist (7th Int. Congress of Endocrinology July 1-7, 1984, Quebec City, Canada ; Excerpta Medica, Amsterdam-Oxford-Princeton ; Drugs of the Future 9, 755, 1984).

Als weiteres erfindungsgemäßes Beispiel sei 11β-(4-Dimethylaminophenyl)-17α-(but-1-in-3-enyl)-17β-hydroxy-4,9-estradien-3-on (D) angeführt ; diese Verbindung zeigt im Antigestagen-Test eine mit RU 38.486 (B) vergleichbare Wirkung, im TAT-Test auf antiglucocorticoide Wirkung ist sie stärker wirksam als RU 38.486 (B).

Der Anti-Thymolyse-Test ist ein in-vivo-Test an der Ratte auf antiglucocorticoide Wirkung. Er basiert darauf, daß Glucocorticoide eine Suppression des Thymus induzieren. Von Substanzen mit antiglucocorticoider Wirkung ist die Aufhebung oder Reduzierung dieses Corticoideffektes zu erwarten. In diesem Test erhalten männliche adrenalektomierte Ratten über 4 Tage die zu prüfenden Substanzen in Kombination mit einer wirksamen Standard-Dexamethasondosis von 0,01 mg/d s. c. injiziert. Am 5. Tag erfolgt die Autopsie. Man ermittelt dann das Thymusgewicht. Zur Beurteilung der antiglucocorticoiden Wirkung der Prüfsubstanz wird die Differenz der Thymus-Gewichte zwischen Lösungsmittelkontrolle

(Benzylbenzoat-Rizinusöl-Gemisch im Verhältnis 1 + 4) und der Gruppe, die allein Dexamethason erhält, gleich 100 % gesetzt. Es wird eine mittlere prozentuale antiglucocorticoide Wirkung ( = Aufhebung der Dexamethasoninduzierten Thymus-Suppression in %) berechnet.

Wie Abbildung 1 und Tabelle 2 zeigen, ist die beispielsweise genannte erfindungsgemäße Verbindung A im in-vivo-Anti-Thymolyse-Test deutlich stärker antiglucocorticoid wirksam als die als Standard anzusehende Verbindung RU 38.486 (B).

Tabelle 2

Aufhebung der Dexamethason-induzierten Thymus-Suppression

| Behandlung: Dexamethason (mg/d s.c.) | Behandlung: Substanz (mg/d s.c.) | | rel. Thymus-Gewicht (mg/100 g Körpergewicht) Mittelwert | Aufhebung des Corticoid-Effekts in % |
|---|---|---|---|---|
| - | - | - | 331,0 | - |
| 0,01 | | - | 86,7 | - |
| 0,01 | | 3,0 | 130,6 | 18,0 |
| 0,01 | A | 10,0 | 235,5 | 60,9 |
| 0,01 | | 30,0 | 309,0 | 91,0 |
| | | - | 385,4 | |
| 0,01 | | - | 87,2 | |
| 0,01 | | 3,0 | 125,6 | 12,9 |
| 0,01 | B | 10,0 | 178,1 | 30,5 |
| 0,01 | | 30,0 | 264,7 | 59,5 |

Die Erfindung betrifft auch pharmazeutische Präparate, die Verbindungen der allgemeinen Formel I enthalten.

Die pharmakologisch wirksamen erfindungsgemäßen Verbindungen der allgemeinen Formel I können nach an sich bekannten Methoden der Galenik zu pharmazeutischen Präparaten für die enterale, perkutane oder parenterale Applikation verarbeitet werden.

Die Dosierung der erfindungsgemäßen Verbindungen liegt beim Menschen bei etwa 1 bis 1 000 mg, vorzugsweise 5 bis 200 mg, pro Tag.

(Siehe Tabelle Seite 5 f.)

Beeinflussung der Dexamethason-induzierten Thymus-Suppression durch
11β-(4-Dimethylaminophenyl)-17α-methoxymethyl-17β-hydroxy-4,9-estradien-3-on (A)

Behandlung adrenalektomierter ♂-Ratten im Gewicht von 100-130 g über 4 Tage (Dosis in mg/d s.c.);
Ermittlung der Thymus-Gewichte an Tag 5. - n = 6 Tiere/ Gruppe

| = 95 % Konfidenzintervall für %-Aufhebung

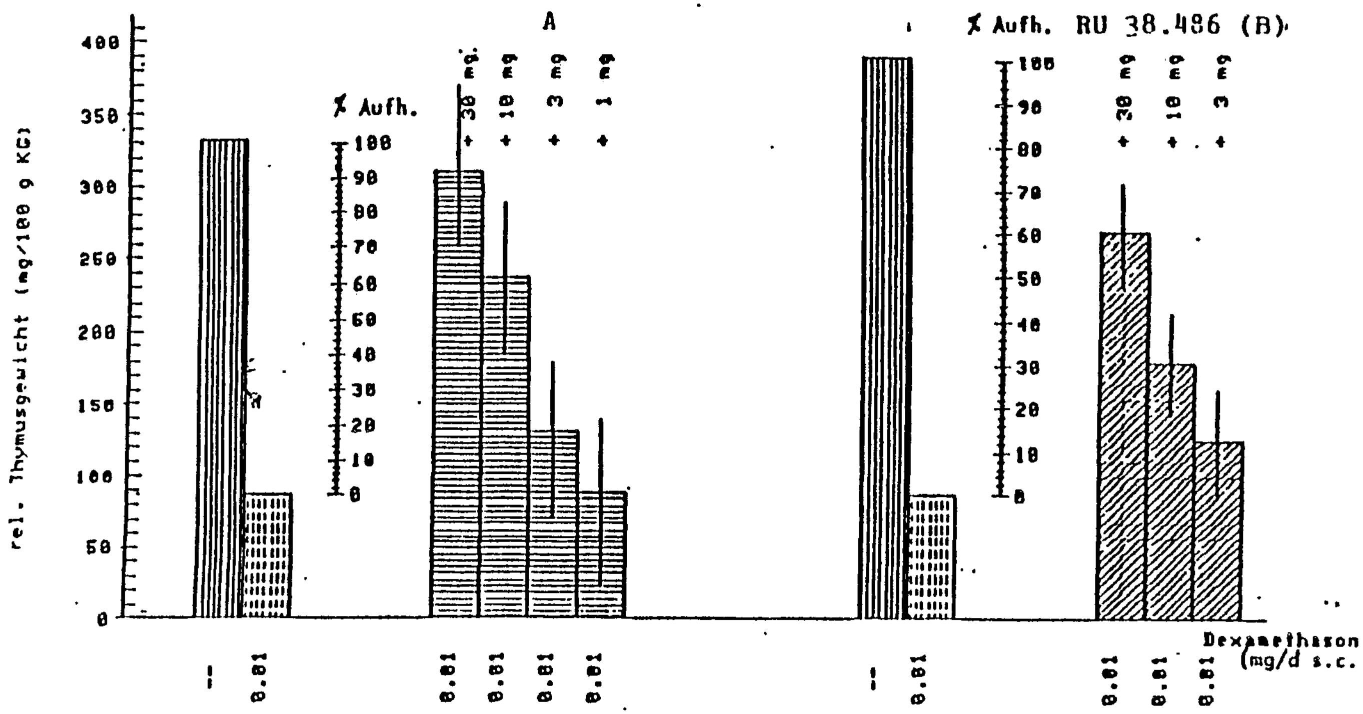

Die in $R^3$ der allgemeinen Formel I enthaltenen Alkyl- bzw. Acylgruppen sollen jeweils 1 bis 4 Kohlenstoffatome enthalten, wobei die Methyl- und Ethyl- bzw. die Formyl-, Acetyl- und Propionylgruppe bevorzugt sind.

Die neuen 13-Alkyl-11β-phenylestradiene der allgemeinen Formel I werden erfindungsgemäß hergestellt, indem man eine Verbindung der allgemeinen Formel II

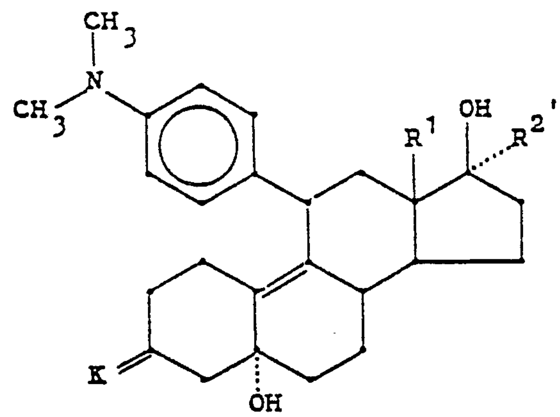

(II)

worin K eine in Form des Ketals, des Thioketals, des Oxims oder des Methyloxims blockierte Ketogruppe bedeutet, $R^1$ die oben genannte Bedeutung hat und $R^{2'}$ die gleiche Bedeutung wie $R^2$ hat, wobei gegebenenfalls vorhandene Hydroxy- oder Mercaptogruppen gesehützt sind, der Einwirkung eines Dehydratisierungsmittels, das auch zur Freisetzung der geschützten Funktion befähigt ist, zur Wasserabspaltung unter gleichzeitiger Ausbildung der 4(5)-Doppelbindung unterwirft und gewünschtenfalls eine in $R^2$ vorhandene Hydroxy- oder Mercaptogruppe unter Bildung des Produkts der allgemeinen Formel I mit X in der Bedeutung eines Sauerstoffatoms verethert bzw. verestert und gewünschtenfalls anschließend mit Hydroxylamin-hydrochlorid in Gegenwart von tertiären Aminen bei Temperaturen zwischen —20 und +40 °C umsetzt.

Ausgehend von den Verbindungen der allgemeinen Formel II wird zur Wasserabspaltung unter Ausbildung der 4(5)-Doppelbindung und zur gleichzeitigen Entfernung vorhandener Schutzgruppen mit Säure oder einem sauren Ionenaustauscher behandelt. Die saure Behandlung erfolgt in an sich bekannter Weise, indem man die Verbindung der Formel II, die zumindest eine Schutzgruppe enthält, in einem mit Wasser mischbaren Lösungsmittel, wie wäßrigem Methanol, Ethanol oder Aceton, löst und auf die Lösung katalytische Mengen Mineral- oder Sulfonsäure, wie Salzsäure, Schwefelsäure, Phosphorsäure, Perchlorsäure oder p-Toluolsulfonsäure, oder eine organische Säure, wie Essigsäure, so lange einwirken läßt, bis Wasser abgespalten ist und die Schutzgruppe(n) entfernt ist(sind). Die Umsetzung, die bei Temperaturen von 0 bis 100 °C abläuft, kann auch mit einem sauren Ionenaustauscher vorgenommen werden. Der Verlauf der Umsetzung kann mit analytischen Methoden, beispielsweise durch Dünnschichtchromatographie entnommener Proben, verfolgt werden.

Die in der allgemeinen Formel II von K und $R^{2'}$ umfaßten Schutzgruppen sind im sauren Milieu leicht abspaltbare Gruppen, wie zum Beispiel die Ethylendioxyketal-, Ethylendithioketal-, 2,2-Dimethyltrimethylendioxyketal-, Hydroxyimino-, Methoxyimino-, Tetrahydropyranyl-, Methoxymethyl- oder Ethoxymethylgruppe.

Die so erhaltenen Verbindungen der allgemeinen Formel I mit X in der Bedeutung eines Sauerstoffatoms können gewünschtenfalls durch Umsetzung mit Hydroxylaminhydrochlorid in Gegenwart von tertiären Aminen bei Temperaturen zwischen —20 und +40 °C in die Oxime (Formel I mit X in der Bedeutung der Hydroxyiminogruppierung N~OH, wobei die Hydroxygruppe syn- oder antiständig sein kann) überführt werden. Geeignete tertiäre Basen sind beispielsweise Trimethylamin, Triäthylamin, Pyridin, N,N-Dimethylaminopyridin, 1,5-Diazabicyclo [4.3.0]-nonen-5 (DBN) und 1,5-Diazabicyclo [5.4.0] undecan-5 (DBU), wobei Pyridin bevorzugt ist.

Wird eine Veresterung bzw. Veretherung von Verbindungen der allgemeinen Formel I, deren Substituent $R^2$ eine Hydroxy- oder Mercaptogruppe enthält, gewünscht, so erfolgt diese Acylierung bzw. Veretherung in an sich bekannter Weise. Die Veresterung erfolgt beispielsweise durch Umsetzung mit dem Säureanhydrid in Pyridin bei Raumtemperatur. Die Veretherung kann zum Beispiel mit Methyljodid in Gegenwart einer Base, wie beispielsweise n-Butyllithium, erfolgen.

Die Herstellung der Ausgangsverbindungen der allgemeinen Formel II geht, wie zum Beispiel in den europäischen Patentanmeldungen 84101721.3 und 82400025.1 beschrieben, aus vom Epoxid der allgemeinen Formel III

(Siehe Formel III Seite 7 f.)

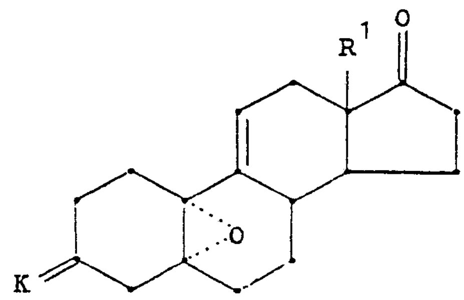

(III)

Die Einführung des 11β-Phenylrestes unter Ausbildung des $\Delta^{9,10}$-5α-Hydroxy-Strukturelements erfolgt entweder durch Cu(I)-katalysierte Grignard-Reaktion mit den entsprechenden Arylmagnesiumhalogeniden (Tetrahedron Letters 1979, 2051) oder durch Umsetzung mit gemischten Organocupraten der Formel $R_2Cu(CN)Li_2$ (J. Amer. Chem. Soc. 103 (1981) 7672).

Die Einführung des Substituenten $R^2$ erfolgt nach den üblichen Verfahren des $C_{17}$-Seitenkettenaufbaus durch nucleophile Addition an das C-17-Keton bzw. an das daraus herstellbare 17-Spirooxiran der allgemeinen Formel IV

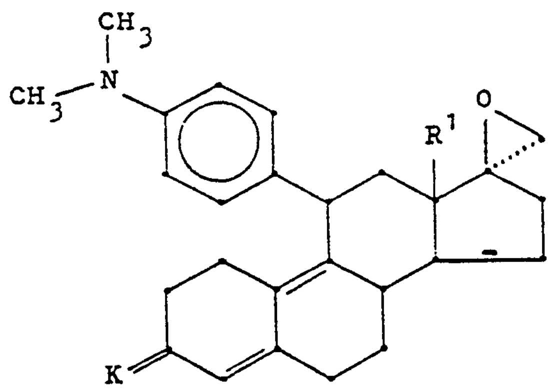

(IV)

[M. Hübner et al, Journal f. prakt. Chemie 314, 667 (1972), M. Hübner et al, DOS 23 35 143 (1973), Arzneim. Forsch. 30, 401 (1980)] mit K in der angegebenen Bedeutung und gegebenenfalls Folgereaktionen (« Terpenoids and Steroids », Specialist Periodical Report, The Chemical Society, London, Vol. 1-12).

Die Öffnung des Spirooxirans zu den entsprechenden 17α-substituierten Derivaten erfolgt nach an sich bekannten Methoden (s. z. B. K. Ponsold et al W.P. DDR 72259 [1968], K. Ponsold et al, Z. Chem. 11, 106 [1971]), z. B. durch Umsetzung mit Natriumethylat (zur 17α-Ethoxymethyl-Verbindung) oder z. B. mit Natriumthioethylat (zur 17α-Ethylthiomethyl-Verbindung).

Die Einführung der But-1-in-3-enyl-Seitenkette in die 17α-Position kann durch Reaktion des 17-Ketons mit Butinyl-THP-ether in Gegenwart einer Base, wie zum Beispiel Kalium-t-butylat, unter gleichzeitiger Abspaltung von 2-Hydroxy-tetrahydropyran erfolgen.

Die Einführung von 3-Hydroxypropin, -propen bzw. -propan in 17-Stellung erfolgt durch Umsetzung des 17-Ketons mit metallierten Derivaten des Propargylalkohols, zum Beispiel mit 1-Lithium-3-tetrahydropyran-2'-yloxy-propin-1, zu den 17-(3-Hydroxy-1-propinyl)-17-hydroxy-Verbindungen, die anschließend zu den 17-(3-Hydroxypropyl- bzw. 3-Hydroxypropenyl)-17-hydroxy-Verbindungen hydriert werden. Die Hydrierung muß unter Bedingungen durchgeführt werden, die ausschließlich den Angriff an der C-C-Dreifachbindung gewährleisten, ohne die gegebenenfalls vorhandene tetrasubstituierte 9(10)-Doppelbindung abzusättigen. Das gelingt zum Beispiel bei der Hydrierung bei Raumtemperatur und Normaldruck in Lösungsmitteln wie Methanol, Ethanol, Propanol, Tetrahydrofuran (THF) oder Essigster unter Zusatz von Edelmetall-Katalysatoren wie Platin oder Palladium.

Die Einführung der homologen Hydroxyalkin-, Hydroxyalken- und Hydroxyalkangruppen erfolgt in entsprechender Weise mit Homologen des Propargylalkohols.

Die Verbindung mit der Z-konfigurierten Doppelbindung in der Hydroxypropenylgruppe entsteht durch Hydrieren der acetylenischen Dreifachbindung mit einem desaktivierten Edelmetallkatalysator (J. Fried, J. A. Edwards : Organic Reactions in Steroid Chemistry, Van Nostrand Reinhold Company 1972, Seite 134, und H.O. House : Modern Synthetic Reactions 1972, Seite 19). Als desaktivierte Edelmetallkatalysatoren kommen beispielsweise 10 % Palladium auf Bariumsulfat in Gegenwart eines Amins oder 5 % Palladium auf Calciumcarbonat unter Zusatz von Blei(II)-acetat infrage. Die Hydrierung wird nach der Aufnahme von einem Äquivalent Wasserstoff abgebrochen.

Die Verbindung mit der E-konfigurierten Doppelbindung in der Hydroxypropenylgruppe entsteht durch Reduktion der acetylenischen Dreifachbindung in an sich bekannter Weise. In der Literatur sind

eine ganze Reihe von Methoden zur Umwandlung von Alkinen in trans-Olefine beschrieben, beispielsweise die Reduktion mit Natrium in flüssigem Ammoniak (J. Am. Chem. Soc. 63 (1941) 216), mit Natriumamid in flüssigem Ammoniak (J. Chem. Soc. 1955, 3558), mit Lithium in niedermolekularen Aminen (J. Am. Chem. Soc. 77 (1955) 3378), mit Boranen (J. Am. Chem. Soc. 93 (1971) 3395 und 94 (1972) 6560), mit Diisobutylaluminiumhydrid und Methyl-Lithium (J. Am. Chem. Soc. 89 (1967) 5085) und insbesondere mit Lithiumaluminiumhydrid/Alkoholat (J. Am. Chem. Soc. 89 (1967) 4245). Eine weitere Möglichkeit ist die Reduktion der Dreifachbindung mit Chrom(II)-sulfat in Gegenwart von Wasser oder Dimethylformamid in schwach saurem Milieu (J. Am. Chem. Soc. 86 (1964) 4358) sowie allgemein die Reduktion durch Einwirkung von Übergangsmetallverbindungen unter Wechsel der Oxydationsstufe.

Beispiel 1

11β-(4-Dimethylaminophenyl)-17α-(but-1-in-3-enyl)-17β-hydroxy-4,9(10)-estradien-3-on

1,1 g 11β-(4-Dimethylaminophenyl)-17α-(but-1-in-3-enyl)-3,3-(2,2-dimethylpropan-1,3-dioxy)-9(10)-estren-5α-17β-diol werden in 7 ml 70 %iger Essigsäure eine Stunde bei 45 °C gerührt. Anschließend wird die wäßrige Phase mit gesättigter $NaHCO_3$-Lösung neutralisiert und mit Methylenchlorid extrahiert. Trocknen der vereinigten organischen Phasen über $Na_2SO_4$, Abzug des Solvens im Vakuum und Chromatographie des Rückstands an Kieselgel mit Hexan/Essigester liefern 633 mg (71 %) der Titelverbindung.

IR (KBr) : 3 440 $cm^{-1}$ OH
1 655 } 1 615 } unges. Keton
1 520 Aromat

$^1$H-NMR (TMS als interner Standard ; ($CHCl_3$) :
δ (ppm) 0,58 (3H,s,H-18) ; 2,92 ((H,s,N($CH_3$)$_2$) ;
4,25-4,45 (1H,m,H-11) ;
5,35-6,05 (3H,m,H-olefin.) ; 5,75 (1H,s,H-4) ;
6,85 (4H,dd,$J_1$ = 9 und $J_2$ = 33Hz, H-aromat.).

Das Ausgangsmaterial wird auf folgendem Wege hergestellt :

Zu einer Lösung von 987 mg (2 mmol) 11β-(4-Dimethylaminophenyl)-3,3-(2,2-dimethylpropan-1,3,-dioxy)-(5-hydroxy-9(10)-estren-17-on in 20 ml abs. Tetrahydrofuran (THF) werden 1,64 ml (10 mmol) Butinyl-THP-Ether und 2,24 g (20 mmol) Kaliumtert. butylat bei Raumtemperatur zugegeben. Nach dreistündigem Rühren wird das Reaktionsgemisch auf eine Mischung aus 10 ml 2n HCl und 40 ml ges. $NH_4Cl$-Lösung gegossen und die wäßrige Phase mit Methylenchlorid extrahiert. Die vereinigten organischen Phasen werden über.

$Na_2SO_4$ getrocknet und am Vakuum eingeengt. Chromatographie an Alox (Stufe III, neutral) mit Hexan/Essigester führen zu 1,191 g (92 %) des gewünschten Produkts.

Beispiel 2

11β-(4-Dimethylaminophenyl)-17α-(methoxymethyl)-17β-hydroxy-4,9(10)-estradien-3-on

1,024 g (1,9 mmol) 11β-(4-Dimethylaminophenyl)-17α-methoxymethyl-3,3-(2,2-dimethylpropan-1,3-dioxy)-9(10)-estren-5α,17β-diol werden wie unter Beispiel 1 beschrieben umgesetzt. Man erhält 900 mg Rohprodukt. Eine Chromatographie über Kieselgel mit Hexan/Essigester ergeben 669 mg (78 %) der gewünschten Endverbindung.

$[a]^{25}_D$ = 203,5° (c = 1,03 ; $CHCl_3$).

Das Ausgangsmaterial wird auf folgendem Wege hergestellt :

a) 11β-(4-Dimethylaminophenyl)-5α-hydroxy-3,3-(2,2-dimethylpropan1,3-dioxy)-9(10)-estren-17β-spiro-1′,2′-oxiran

2,6 g (5,3 mmol) 11β-(4-Dimethylaminophenyl)-3,3-(2,2-dimethylpropan-1,3-dioxy)-5α-hydroxy-9(10)-estren-17-on werden in 50 ml abs. Dimethylformamid unter Schutzgas gelöst und nacheinander mit 2,15 g Trimethylsulfoniumjodid (10,6 mmol) und 1,18 g (10,6 mmol) Kaliumtert.-butylat versetzt. Nach 16stündigem Nachrühren bei Raumtemperatur wird das Reaktionsgemisch auf 110 ml ges. $NaHCO_3$-Lösung gegossen, die organische Phase abgetrennt und die wäßrige Phase mit Essigester extrahiert. Nach Trocknen der vereinigten organischen Phasen über $Na_2SO_4$ und Abzug der Solvenzien am Vakuum werden 2,67 g Rohprodukt isoliert. Chromatographie an Alox (Stufe III, neutral) mit Hexan/Essigester führt zu 2,43 g (91 %) der gewünschten Verbindung.

IR (KBr) : 3 520 $cm^{-1}$ OH
1 615 } 1 520 } Aromat

$^1H$-NMR (TMS als interner Standard ; $CHCl_3$) :
δ (ppm) 0,5 (3H,s,H-18) ; 0,85 (3H,s,$CH_3$-Ketal) ;
1,02 (3H,s,$CH_3$-Ketal) ; 2,57 (1H,d,J = 5Hz, H-Epoxid) ; 2,92 (1H,d,J = 5Hz,H-Epoxid) ;
2,89 (6H,s,2 x $NCH_3$) ;
3,4-3,6 (4H,m,$OCH_2$) ;
4,05-4,25 (1H,m,H-11) ;
4,37 (1H,s,tert. OH) ;
6,8 (4H,dd,$J_1$ = 6 und $J_2$ = 34Hz, aromat.-H).

b) 11β-(4-Dimethylaminophenyl)-17α-(methoxymethyl)-3,3-(2,2-dimethylpropan-1,3-dioxy)-9(10)-estren-5α, 17β-diol

1 g (1,97 mmol) Epoxid werden in 10 ml abs. Methanol gelöst und mit 20 ml einer 3 m methanolischen Natriummethanolatlösung versetzt. Nach 3 stündigem Rühren bei Raumtemperatur wird das Reaktionsgemisch auf 150 ml gesättigte $NH_4Cl$-Lösung gegossen und die wäßrige Phase mit Methylenchlorid extrahiert. Waschen der vereinigten organischen Phasen mit Wasser, Trocknen über $Na_2SO_4$ und Abzug des Solvens am Vakuum führen zu 1,024 g (96 %) Rohprodukt.

Beispiel 3

11β-(4-Dimethylaminophenyl)-17-(2-methoxyethyl)-17β-hydroxy-4,9(10)-estradien-3-on

632 mg (1,14 mmol) 11β-(4-Dimethylaminophenyl)-17-(2-methoxyethyl)-3,3-(2,2-dimethylpropan-1,3-dioxy)-9(10)-estren-5α,17β-diol werden wie unter Beispiel 1 beschrieben umgesetzt. Chromatographie an Kieselgel mit Hexan/Essigester ergeben 285 mg (56 %) der gewünschten Endverbindung.
IR (KBr) : 3 480 $cm^{-1}$ OH
1 660 }
1 610 } unges. Keton
1 515 Aromat
$^1H$-NMR (TMS als interner Standard ; $CHCl_3$) :
δ (ppm) 0,59 (3H,s,H-18) ; 2,9 (6H,s,$N(CH_3)_2$) ;
3,35 (3H,s,$OCH_3$) ; 3,43 (1H,s,OH) ;
3,6-3,72 (2H,m,O-$CH_2$) ;
4,32 (1H,d,J = 6,5Hz,H-11) ; 5,73 (1H,s,H-4) ;
6,8 (4H,dd,$J_1$ = 9 und $J_2$ = 34Hz, H-aromat.).
Das Ausgangsmaterial wird auf folgendem Wege hergestellt :

a) 17α-(tert.-Butoxycarbonylmethyl)-11β-(4-dimethylaminophenyl)-3,3-(2,2-dimethylpropan-1,3-dioxy)-9(10)- estren-5α,17β-diol

4,09 ml (30, 38 mmol) Essigsäuretert.-butylester werden bei —78 °C zu einer Lösung von 31,9 mmol Lithiumdiisopropylamid in 150 ml abs. Tetrahydrofuran zugetropft. Nach 1 stündigem Nachrühren wird die Reaktionslösung auf —60 °C erwärmt und eine Lösung von 3 g (6,08 mmol) 11β-(4-Dimethylaminophenyl)-3,3-(2,2-dimethylpropan-1,3-dioxy)-5α-hydroxy-9(10)-estren-17-on in 50 ml abs. Tetrahydrofuran langsam zugegeben. Nach beendeter Zugabe wird 1 Stunde nachgerührt, bevor das Reaktionsgemisch mit 100 ml wäßriger Ammoniumchloridlösung versetzt wird. Die wäßrige Phase wird mit Methylenchlorid extrahiert ; nach Trocknen der vereinigten organischen Phasen mit Natriumsulfat und Abzug der Solvenzien im Vakuum wird der Rückstand über eine Aloxsäule (neutral, Stufe III) mit Hexan/Essigester chromatographiert. Es werden 2,67 g des gewünschten Produkts (72 %) erhalten.

b) 11β-(4-Dimethylaminophenyl)-17α-(2-hydroxyethyl)-3,3-(2,2-dimethylpropan-1,3-dioxy)-9(10)-estren-5α, 17β-diol

1,35 g (2,21 mmol) des unter a) erhaltenen tert. Butylesters werden in 30 ml abs. Tetrahydrofuran gelöst und mit 672 mg (10 eq.) Lithiumaluminiumhydrid versetzt. Anschließend wird das Reaktionsgemisch 8 Stunden auf 40 °C erhitzt und über Nacht bei Raumtemperatur nachgerührt. Nach Zugabe von 50 ml Methylenchlorid wird die Reaktionslösung auf 0 °C gekühlt und so lange mit gesättigter Natriumhydrogencarbonatlösung versetzt, bis ein flockiger Niederschlag entsteht. Die überstehende flüssige Phase wird abdekantiert und der Niederschlag unter kräftigem Rühren mehrmals mit Methylenchlorid ausgewaschen. Nach Abzug der Solvenzien im Vakuum wird der Rückstand über $Al_2O_3$ (neutral, Stufe III) mit Essigester/Ethanol chromatographiert. Es werden 1,066 g der gewünschten Verbindung (89 %) erhalten.

c) 11β-(4-Dimethylaminophenyl)-17-(2-methoxyethyl)-3,3-(2,2-dimethylpropan-1,3-dioxy)-9(10)-estren-5α, 17β-diol

1,06 g (1,97 mmol) der unter b) erhaltenen Substanz werden in 50 ml absolutem Tetrahydrofuran gelöst und bei 0 °C mit 2,9 ml einer 1,5 molaren n-Butyl-lithiumlösung (4,35 mmol) versetzt. Anschließend werden 0,31 ml (5 mmol) Methyljodid langsam zugetropft. Nach 6 stündigem Nachrühren werden 25 ml Methanol zugegeben und das Reaktionsgemisch am Vakuum eingeengt. Nach Zugabe von 100 ml Methylenchlorid wird die organische Phase mit gesättigter $NaHCO_3$-Lösung gewaschen. Trocknen der organischen Phase über $Na_2SO_4$ und Abzug des Solvenz am Vakuum führen zum Rohprodukt. Chromatographie an Alox (neutral, Stufe III) mit Essigester/Hexan ergeben 632 mg (58 %) des gewünschten Ketals.

Beispiel 4

11β-(4-Dimethylaminophenyl)-17α-ethoxymethyl-17β-hydroxy-4,9(10)-estradien-3-on

665 mg (1,2 mmol) 11β-(4-Dimethylaminophenyl)-17α-ethoxymethyl-3,3-(2,2-dimethylpropan-1,3-dioxy)-9-estren-5α,17β-diol werden wie unter Beispiel 1 beschrieben umgesetzt. Nach Chromatographie über Kieselgel mit Hexan/Essigester werden 376 mg (80,4 %) der gewünschten Endverbindung erhalten.
$[\alpha]^{25}_D = 192°$ (c = 0,5 ; $CHCl_3$).

Das Ausgangsmaterial wird auf folgendem Wege hergestellt :

760 mg (1,5 mmol) des nach Beispiel 2a) erhaltenen Epoxids werden in 50 ml abs. Tetrahydrofuran gelöst und mit 23 ml einer 2-molaren ethanolischen Ethanolatlösung versetzt. Anschließend wird 12 Minuten auf 50 °C erhitzt und dann die Lösung über Nacht nachgerührt. Das Reaktionsgemisch wird auf 50 ml gesättigte $NH_4Cl$-Lösung gegossen und die wäßrige Phase mit Methylenchlorid extrahiert. Die vereinigten organischen Phasen werden über $Na_2SO_4$ getrocknet und das Solvens am Vakuum abgezogen. Es werden 829 mg (99,8 %) Rohprodukt erhalten.

Beispiel 5

11β-(4-Dimethylaminophenyl)-17α-(4-hydroxybut-1(Z)-enyl)-17β-hydroxy-4,9(10)-estradien-3-on

Das aus 500 mg (0,887 mmol) Hydroxybutinyladdukt (s. unter 5a)) durch Hydrierung erhaltene Rohprodukt 11β-(4-Dimethylaminophenyl)-17α-(4-hydroxybut-1(Z)-enyl)-3,3-(2,2-dimethylpropan-1,3-dioxy)-9(10)-estren-5α, 17β-diol wird unter den bei Beispiel 1 beschriebenen Bedingungen umgesetzt. Durch Chromatographie des Rückstandes an Kieselgel mit Hexan/Essigester werden 320 mg (78 %) der Titelverbindung erhalten.

IR (KBr) : 3 410 $cm^{-1}$ OH
1 655 }
1 620 } unges. Keton
1 520 Aromat

$^1$H-NMR (TMS als interner Standard ; $CHCl_3$) :
δ (ppm) 0,62 (3H,s,H-18) ; 2,92 (6H,s,$N(CH_3)_2$) ;
3,5-3,85 (2H,m,$O-CH_2$) ;
4,28 (1H,d,J = 6, 5Hz, H-11) ;
5,3-5,8 (2H,m,H-olefin.) ; 5,72 (1H,s,H-4) ;
6,8 (4H,dd,$J_1$ = 9 und $J_2$ = 34Hz, H-aromat.)

Das Ausgangsmaterial wird auf folgendem Wege erhalten :

a) 11β-(4-Dimethylaminophenyl)-17α-(4-hydroxybut-1-inyl)-3,3-(2,2-dimethylpropan-1,3-dioxy)-9(10)-estren-5α, 17β-diol

2,962 g (6 mmol) 11β-(4-Dimethylaminophenyl)-3,3-(2,2-dimethylpropan-1,3-dioxy)-5α-hydroxy-9(10)-estren-17-on werden bei 0 °C in 100 ml abs. Tetrahydrofuran vorgelegt und mit 7,56 g (90 mmol) Kaliumtert.-butylat versetzt. Nach Zutropfen von 3,4 ml (45 mmol) 3-Butin-1-ol wird das Reaktionsgemisch langsam auf 22 °C erwärmt und 16 Stunden bei derselben Temperatur nachgerührt. Anschließend wird es auf ein Gemisch aus 35 ml 2n HCl und 25 ml gesättigter $NH_4Cl$-Lösung gegossen und die wäßrige Phase mit Methylenchlorid extrahiert. Die vereinigten organischen Phasen werden über $Na_2SO_4$ getrocknet und am Vakuum zur Trockne eingeengt. Chromatographie des Rohprodukts an $Al_2O_3$ (neutral, Stufe III) mit Hexan/Essigester führen zu 2,43 g (72 %) des gewünschten Produkts.

b) 11β-(4-Dimethylaminophenyl)-17α-(4-hydroxybut-1(Z)-enyl)-3,3-(2,2-dimethylpropan-1,3-dioxy)-9(10)-estren-5α, 17β-diol

500 mg (0,887 mmol) des Hydroxybutinyladdukts werden in 20 ml Methanol gelöst und sukkzessive mit 1,1 ml Triethylamin und 50 mg Palladium auf $BaSO_4$ (10 %) versetzt. Die Hydrierung unter Normaldruck wird nach Aufnahme eines Äquivalents Wasserstoff abgebrochen. Filtration und Einengen des Filtrats bis zur Trockne führen zu dem gewünschten Rohprodukt.

Beispiel 6

11β-(4-Dimethylaminophenyl)-17α-(2-hydroxyethyl)-17β-hydroxy-4,9(10)-estradien-3-on

1 g 11β-(4-Dimethylaminophenyl)-17α-(2-hydroxyethyl)-3,3-(2,2-dimethylpropan-1,3-dioxy)-9(10)-estren-5α, 17β-diol wird unter den bei Beispiel 1 beschriebenen Bedingungen zum Dienon gespalten. Nach Chromatographie des Rohprodukts an Kieselgel mit Essigester/Ethanol erhält man 610 mg (75 %) der Titelverbindung.

IR (KBr) : 3 420 $cm^{-1}$ OH
1 660, 1 610 } unges. Keton

$^1$H-NMR (TMS als interner Standard ; $CHCl_3$) :
δ (ppm) 0,58 (3H,s,H-18) ; 2,93 (6H,s,$N(CH_3)_2$) ;
3,75-4,1 (2H,m,$O-CH_2$) ;
4,35 (1H,d,J = 6,5Hz,H-11) ; 5,74 (1H,s,H-4) ;
6,82 (4H,dd,$J_1$ = 9 und $J_2$ = 33Hz,H-aromat.).

Das Ausgangsmaterial ist die als Beispiel 3b) beschriebene Verbindung.

Beispiel 7

11β-(4-Dimethylaminophenyl)-17α-ethylthiomethyl-17β-hydroxy-4,9(10)-estradien-3-on

774 mg 11β-(4-Dimethylaminophenyl)-17α-ethylthiomethyl-3,3-(2,2-dimethylpropan-1,3-dioxy)-9(10)-estren-5α-17β-diol werden unter den in Beispiel 1 beschriebenen Bedingungen umgesetzt. Nach Chromatographie des Rohprodukts an Kieselgel mit Hexan/Essigester werden 312 mg (47 %) der Titelverbindung erhalten.

$[\alpha]^{25}_D = 163°$ (c = 0,5, $CHCl_3$).

Das Ausgangsmaterial wird auf folgendem Wege erhalten :

1,02 g (2 mmol) des nach Beispiel 2a) erhaltenen Epoxids werden in 40 ml abs. THF gelöst und mit 2,22 ml Ethanthiol (30 mmol) versetzt. Nach Abkühlen der Lösung auf 5 °C werden 2,24 g (20 mmol) KOtBu zugegeben und 14 Stunden bei Raumtemperatur nachgerührt. Anschließend wird das Reaktionsgemisch auf 25 ml gesättigte $NH_4Cl$-Lösung gegossen und die wäßrige Phase vorsichtig mit-1 n HCl-Lösung auf pH 8 gebracht. Extraktion mit Methylenchlorid, Trocknen der organischen Phasen über $Na_2SO_4$ und Abzug des Solvens am Vakuum führen zu 1,13 g Rohprodukt. Chromatographie über Kieselgel mit Hexan/Essigester ergeben 774 mg (68 %) der gewünschten Verbindung.

Beispiel 8

11β-(4-Dimethylaminophenyl)-17α-(methoxymethyl)-17β-hydroxy-4,9-estradien-3-on-anti-oxim

und

Beispiel 9

11β-(4-Dimethylaminophenyl)-17α-(methoxymethyl)-17β-hydroxy-4,9-estradien-3-on-syn-oxim

Eine Lösung von 1,5 g des nach Beispiel 2 erhaltenen 11β-(4-Dimethylaminophenyl)-17α-(methoxymethyl)-17β-hydroxy-4,9-estradien-3-ons in 20 ml Pyridin wird unter Eiswasserkühlung portionsweise mit 980 mg Hydroxylaminhydrochlorid versetzt. Nach Zugabe rührt man 30 Minuten bei + 5 °C, gießt dann in eine Mischung aus Eiswasser/0,5 n-HCl und extrahiert mit Methylenchlorid. Das Rohprodukt wird an Kieselgel mit Hexan/Essigester chromatographiert und

a) 820 mg 11β-(4-Dimethylaminophenyl)-17α-(methoxymethyl)-17β-hydroxy-4,9-estradien-3-on-anti-oxim [UV (MeOH) = 288 (27400)]

b) 320 mg 11β-(4-Dimethylaminophenyl)-17α-(methoxymethyl)-17β-hydroxy-4,9-estradien-3-on-syn-oxim [UV (MeOH) = 289 (28200)]

isoliert.

Beispiel 10

11β-(4-Dimethylaminophenyl)-17α-(but-1-in-3-enyl)-17β-hydroxy-4,9-estradien-3-on-anti-oxim

und

Beispiel 11

11β-(4-Dimethylaminophenyl)-17α-(but-1-in-3-enyl)-17β-hydroxy-4,9-estradien-3-on-syn-oxim

Nach der Vorschrift für die Beispiele 8 und 9 werden aus 1,5 g 11β-(4-Dimethylaminophenyl)-17α-(but-1-in-3-enyl)-17β-hydroxy-4,9-estradien-3-on (Beispiel 1) nach Aufarbeitung und Chromatographie

a) 905 mg 11β-(4-Dimethylaminophenyl)-17α-(but-1-in-3-enyl)-17β-hydroxy-4,9-estradien-3-on-anti-oxim [UV (MeOH) = 288 (27100)]

b) 310 mg 11β-(4-Dimethylaminophenyl)-17α-(but-1-in-3-enyl)-17β-hydroxy-4,9-estradien-3-on-syn-oxim [UV (MeOH) = 290 (27500)]

isoliert.

**Patentansprüche** (für die Vertragsstaaten : BE, CH, LI, DE, FR, GB, IT, LU, NL, SE)

1. 11β-Aryl-Estradiene der allgemeinen Formel I

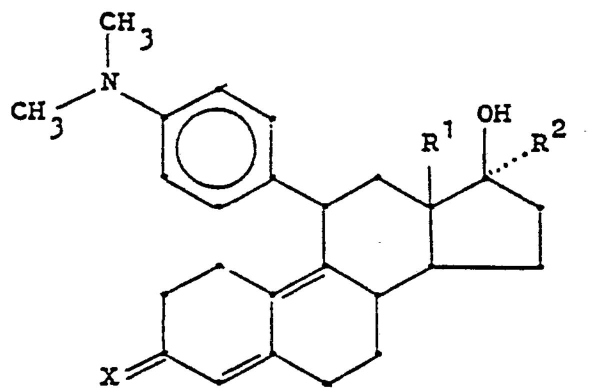

(I)

worin

X ein Sauerstoffatom oder eine Hydroxyiminogruppe N ~ OH, wobei die Hydroxygruppe syn- oder antiständig sein kann,

$R^1$ eine Methyl- oder Ethylgruppe,

$R^2$ eine $—C≡C—CH=CH_2$, eine $—CH_2—(CH_2)_n—Y—R^3$ oder eine $—CH=CH—(CH_2)_m—Y—R^3$-Gruppe

bedeuten, wobei Y für ein Sauerstoff- oder Schwefelatom, n für die Ziffern 0 oder 1, m für die Ziffern 1, 2 oder 3 und $R^3$ für ein Wasserstoffatom, für einen Alkyl- oder Acylrest mit jeweils 1 bis 4 Kohlenstoffatomen steht, mit der Maßgabe, daß wenn m für 1 und Y für ein Sauerstoffatom stehen, $R^3$ ein Alkylrest mit 1 bis 4 Kohlenstoffatomen bedeutet.

2. 11β-(4-Dimethylaminophenyl)-17α-(but-1-in-3-enyl)-17β-hydroxy-4,9-estradien-3-on.
11β-(4-Dimethylaminophenyl)-17α-(methoxymethyl)-17β-hydroxy-4,9-estradien-3-on.
11β-(4-Dimethylaminophenyl)-17α-(2-methoxyethyl)-17β-hydroxy-4,9-estradien-3-on.
11β-(4-Dimethylaminophenyl)-17α-(ethoxymethyl)-17β-hydroxy-4,9-estradien-3-on.
11β-(4-Dimethylaminophenyl)-17α-(4-hydroxybut-1-(Z)-enyl)-17β-hydroxy-4,9-estradien-3-on.
11β-(4-Dimethylaminophenyl)-17α-(2-hydroxyethyl)-17β-hydroxy-4,9-estradien-3-on.
11β-(4-Dimethylaminophenyl)-17α-(ethylthiomethyl)-17β-hydroxy-4,9-estradien-3-on.
11β-(4-Dimethylaminophenyl)-17α-(methoxymethyl)-17β-hydroxy-4,9-estradien-3-on-anti-oxim.
11β-(4-Dimethylaminophenyl)-17α-(methoxymethyl)-17β-hydroxy-4,9-estradien-3-on-syn-oxim.
11β-(4-Dimethylaminophenyl)-17α-(but-1-in-3-enyl)-17β-hydroxy-4,9-estradien-3-on-anti-oxim.
11β-(4-Dimethylaminophenyl)-17α-(but-1-in-3-enyl)-17β-hydroxy-4,9-estradien-3-on-syn-oxim.

3. Verfahren zur Herstellung von Verbindungen der allgemeinen Formel I

(Siehe Formel I Seite 13 f.)

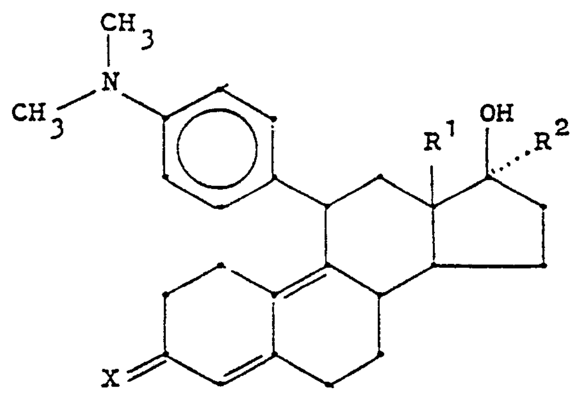

(I)

worin

X ein Sauerstoffatom oder eine Hydroxyiminogruppe N ~ OH, wobei die Hydroxygruppe syn- oder antiständig sein kann,

$R^1$ eine Methyl- oder Ethylgruppe,

$R^2$ eine $-C{\equiv}C-CH{=}CH_2-$, eine $-CH_2-(CH_2)_n-Y-R^3$ oder eine $-CH{=}CH-(CH_2)_m-Y-R^3$-Gruppe

bedeuten, wobei Y für ein Sauerstoff- oder Schwefelatom, n für die Ziffern 0 oder 1, m für die Ziffern 1, 2 oder 3 und $R^3$ für ein Wasserstoffatom, für einen Alkyl- oder Acylrest mit jeweils 1 bis 4 Kohlenstoffatomen steht, mit der Maßgabe, daß wenn m für 1 und Y für ein Sauerstoffatom stehen, $R^3$ ein Alkylrest mit 1 bis 4 Kohlenstoffatomen bedeutet, dadurch gekennzeichnet, daß man eine Verbindung der allgemeinen Formel II

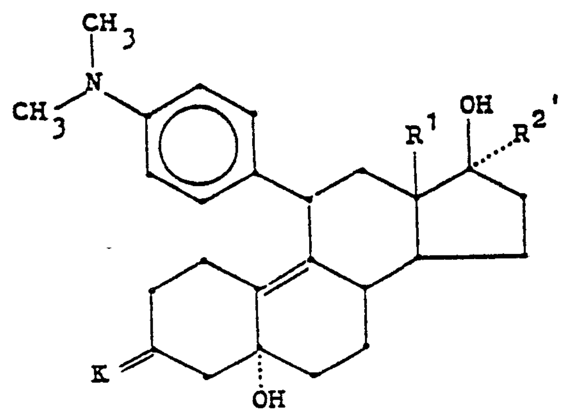

(II)

worin K eine in Form des Ketals, des Thioketals, des Oxims oder des Methyloxims blockierte Ketogruppe bedeutet, $R^1$ die oben genannte Bedeutung hat und $R^{2'}$ die gleiche Bedeutung wie $R^2$ hat, wobei gegebenenfalls vorhandene Hydroxy- oder Mercaptogruppen gesehützt sind, der Einwirkung eines Dehydratisierungsmittels, das auch zur Freisetzung der geschützten Funktion befähigt ist, zur Wasserabspaltung unter gleichzeitiger Ausbildung der 4(5)-Doppelbindung unterwirft und gewünschtenfalls eine in $R^2$ vorhandene Hydroxy- oder Mercaptogruppe unter Bildung des Produkts der allgemeinen Formel I mit X in der Bedeutung eines Sauerstoffatoms verethert bzw. verestert und gewünschtenfalls anschließend mit Hydroxylamin-hydrochlorid in Gegenwart von tertiären Aminen bei Temperaturen zwischen —20 und + 40 °C umsetzt.

4. Pharmazeutische Präparate gekennzeichnet durch einen Gehalt an Verbindungen gemäß den Ansprüchen 1 bis 2.

5. Verwendung von Verbindungen gemäß den Ansprüchen 1 bis 2 zur Herstellung von pharmazeutischen Präparaten.

**Patentanspruch** (für den Vertragsstaat AT)

Verfahren zur Herstellung von Verbindungen der allgemeinen Formel I

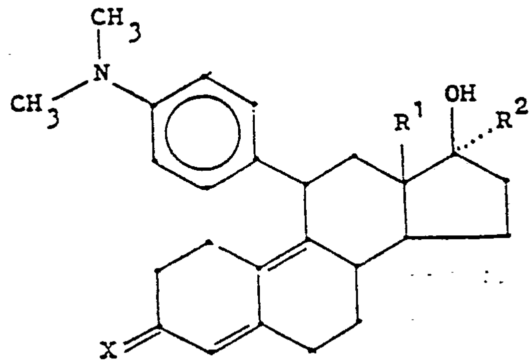

(I)

worin

X ein Sauerstoffatom oder eine Hydroxyiminogruppe N ~ OH, wobei die Hydroxygruppe syn- oder antiständig sein kann,

$R^1$ eine Methyl- oder Ethylgruppe,

$R^2$ eine $—C{\equiv}C—CH{=}CH_2—$, eine $—CH_2—(CH_2)_n—Y—R^3$ oder eine $—CH{=}CH—(CH_2)_m—Y—R^3$-Gruppe

bedeuten, wobei Y für ein Sauerstoff- oder Schwefelatom, n für die Ziffern 0 oder 1, m für die Ziffern 1, 2 oder 3 und $R^3$ für ein Wasserstoffatom, für einen Alkyl- oder Acylrest mit jeweils 1 bis 4 Kohlenstoffatomen steht, mit der Maßgabe, daß wenn m für 1 und Y für ein Sauerstoffatom stehen, $R^3$ ein Alkylrest mit 1 bis 4 Kohlenstoffatomen bedeutet, dadurch gekennzeichnet, daß man eine Verbindung der allgemeinen Formel II

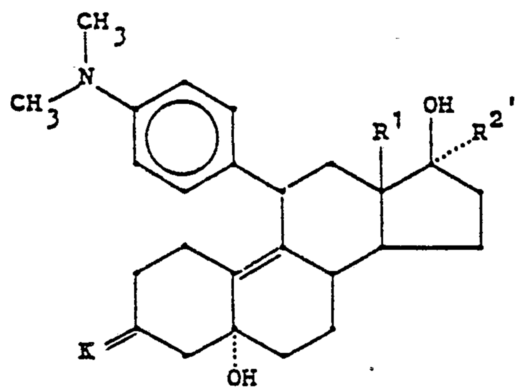

(II)

worin K eine in Form des Ketals, des Thioketals, des Oxims oder des Methyloxims blockierte Ketogruppe bedeutet, $R^1$ die oben genannte Bedeutung hat und $R^{2'}$ die gleiche Bedeutung wie $R^2$ hat, wobei gegebenenfalls vorhandene Hydroxy- oder Mercaptogruppen geschützt sind, der Einwirkung eines Dehydratisierungsmittels, das auch zur Freisetzung der geschützten Funktion befähigt ist, zur Wasserabspaltung unter gleichzeitiger Ausbildung der 4(5)-Doppelbindung unterwirft und gewünschtenfalls eine in $R^2$ vorhandene Hydroxy- oder Mercaptogruppe unter Bildung des Produkts der allgemeinen Formel I mit X in der Bedeutung eines Sauerstoffatoms verethert bzw. verestert und gewünschtenfalls anschließend mit Hydroxylamin-hydrochlorid in Gegenwart von tertiären Aminen bei Temperaturen zwischen — 20 und + 40 °C umsetzt.

**Claims** (for the Contracting States : BE, CH, LI, DE, FR, GB, IT, LU, NL, SE)

1. 11β-aryl-oestradienes of the general formula I

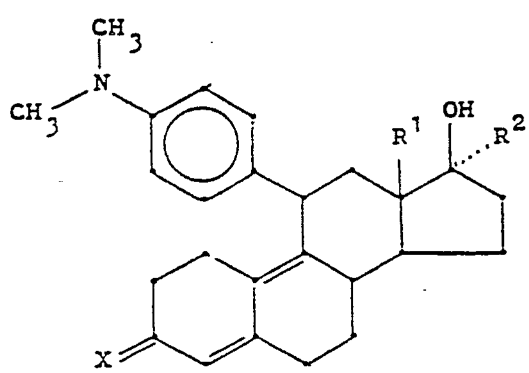

(I)

wherein

X represents an oxygen atom or a hydroxyimino group N ~ OH in which the hydroxy group may be in the syn or anti position,

$R^1$ represents a methyl or ethyl group,

$R^2$ represents a $-C\equiv C-CH=CH_2$, a $-CH_2-(CH_2)_m-Y-R^3$ or a $-CH=CH-(CH_2)_n-Y-R^3$ group,

Y representing an oxygen or sulphur atom, n representing the number 0 or 1, m the number 1, 2 or 3 and $R^3$ a hydrogen atom, or an alkyl or acyl radical each having from 1 to 4 carbon atoms, with the proviso that when m represents 1 and Y represents an oxygen atom, $R^3$ represents an alkyl radical having from 1 to 4 carbon atoms.

2. 11β-(4-dimethylaminophenyl)-17α-(but-1-yn-3-enyl)-17β-hydroxy-4,9-oestradien-3-one,
11β-(4-dimethylaminophenyl)-17α-(methoxymethyl)-17β-hydroxy-4,9-oestradien-3-one,
11β-(4-dimethylaminophenyl)-17α-(2-methoxyethyl)-17β-hydroxy-4,9-oestradien-3-one,
11β-(4-dimethylaminophenyl)-17α-(ethoxymethyl)-17β-hydroxy-4,9-oestradien-3-one,
11β-(4-dimethylaminophenyl)-17α-(4-hydroxybut-1-(Z)-enyl)-17β-hydroxy-4,9-oestradien-3-one,
11β-(4-dimethylaminophenyl)-17α-(2-hydroxyethyl)-17β-hydroxy-4,9-oestradien-3-one,
11β-(4-dimethylaminophenyl)-17α-(ethylthiomethyl)-17β-hydroxy-4,9-oestradien-3-one,
11β-(4-dimethylaminophenyl)-17α-(methoxymethyl)-17β-hydroxy-4,9-oestradien-3-one-anti-oxime,
11β-(4-dimethylaminophenyl)-17α-(methoxymethyl)-17β-hydroxy-4,9-oestradien-3-one-syn-oxime,
11β-(4-dimethylaminophenyl)-17α-(but-1-yn-3-enyl)-17β-hydroxy-4,9-oestradien-3-one-anti-oxime,
11β-(4-dimethylaminophenyl)-17α-(but-1-yn-3-enyl)-17β-hydroxy-4,9-oestradien-3-one-syn-oxime.

3. Process for the manufacture of the compounds of the general formula I

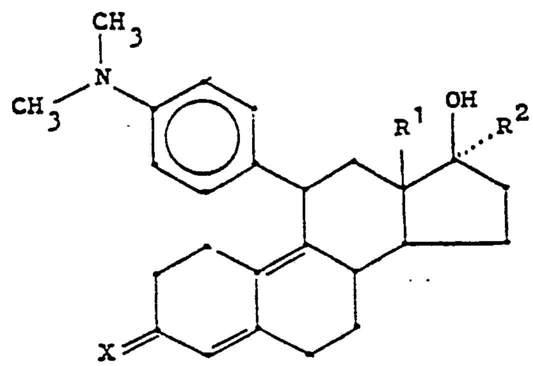

(I)

wherein

X represents an oxygen atom or a hydroxyimino group N~OH in which the hydroxy group may be in the syn or anti position,

$R^1$ represents a methyl or ethyl group,

$R^2$ represents a $-C\equiv C-CH=CH_2-$, a $-CH_2-(CH_2)_n-Y-R^3$ or a $-CH=CH-(CH_2)_m-Y-R^3$ group,

Y representing an oxygen or sulphur atom, n representing the number 0 or 1, m the number 1, 2 or 3 and $R^3$ a hydrogen atom, or an alkyl or acyl radical each having from 1 to 4 carbon atoms, with the proviso that when m represents 1 and Y represents an oxygen atom, $R^3$ represents an alkyl radical having from 1 to 4 carbon atoms,

characterised in that a compound of the general formula II

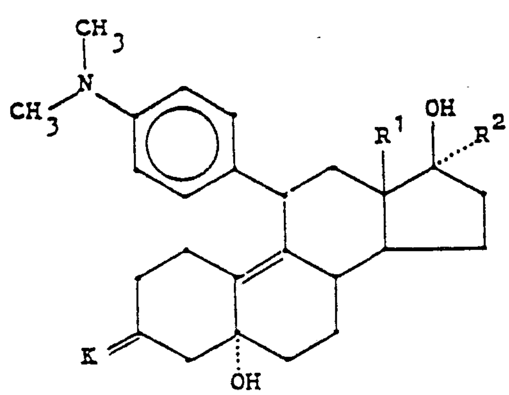

(II)

wherein K represents a keto group blocked in the form of the ketal, thioketal, oxime or methyloxime, $R^1$

has the meaning mentioned above and $R^{2'}$ has the same meaning as $R^2$, any hydroxy or mercapto groups that may be present being protected, is subjected to the action of a dehydrating agent, which is also capable of freeing the protected function, in order to remove the water while simultaneously forming the 4(5) double bond, and, if desired, a hydroxy or mercapto group present in $R^2$ is etherified or esterified to form the product of the general formula I in which X represents an oxygen atom and, if desired, subsequently reacted with hydroxylamine hydrochloride in the presence of a tertiary amine at temperatures of between — 20 °C and + 40 °C.

4. Pharmaceutical preparations, characterised by a content of compounds according to claims 1 to 2.

5. Use of compounds according to claims 1 to 2 for the manufacture of pharmaceutical preparations.

**Claim** (for the Contracting State AT)

1. Process for the manufacture of the compounds of the general formula I

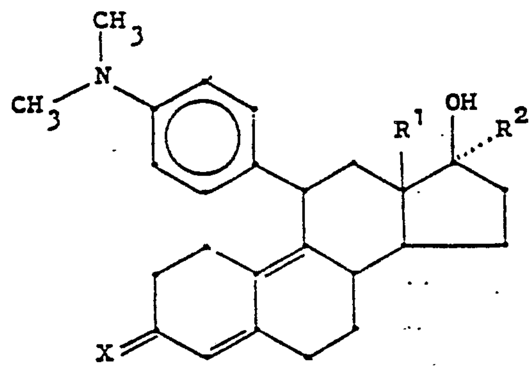

(I)

wherein

X represents an oxygen atom or a hydroxyimino group N~OH in which the hydroxy group may be in the syn or anti position,

$R^1$ represents a methyl or ethyl group,

$R^2$ represents a $—C{\equiv}C—CH{=}CH_2—$, a $—CH_2—(CH_2)_n—Y—R^3$ or a $—CH{=}CH—(CH_2)_m—Y—R^3$ group,
Y representing an oxygen or sulphur atom, n representing the number 0 or 1, m the number 1, 2 or 3 and $R^3$ a hydrogen atom, or an alkyl or acyl radical each having from 1 to 4 carbon atoms, with the proviso that when m represents 1 and Y represents an oxygen atom, $R^3$ represents an alkyl radical having from 1 to 4 carbon atoms,
characterised in that a compound of the general formula II

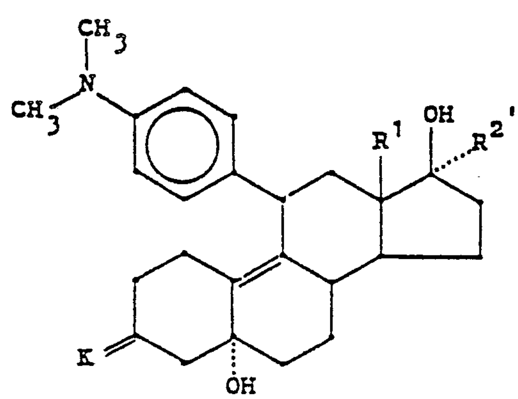

(II)

wherein K represents a keto group blocked in the form of the ketal, thioketal, oxime or methyloxime, $R^1$ has the meaning mentioned above and $R^{2'}$ has the same meaning as $R^2$, any hydroxy or mercapto groups that may be present being protected, is subjected to the action of a dehydrating agent, which is also capable of freeing the protected function, in order to remove the water while simultaneously forming the 4(5) double bond, and, if desired, a hydroxy or mercapto group present in $R^2$ is etherified or esterified to form the product of the general formula I in which X represents an oxygen atom and, if desired, subsequently reacted with hydroxylamine hydrochloride in the presence of a tertiary amine at temperatures of between — 20 °C and + 40 °C.

**Revendications** (pour les Etats contractants : BE, CH, LI, DE, FR, GB, IT, LU, NL, SE)

1. 11β-Aryl-œstradiènes répondant à la formule générale I

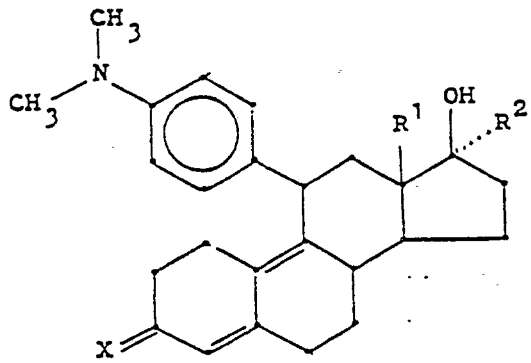

(I)

dans laquelle

X représente un atome d'oxygène ou un groupe hydroxyimino N~OH, où le groupe hydroxy peut être en position syn ou anti,

$R^1$ représente un groupe méthyle ou éthyle,

$R^2$ représente un groupe $—C{\equiv}C—CH{=}CH_2$, $—CH_2—(CH_2)_n—Y—R^3$ ou $—CH{=}CH—(CH_2)_m—Y—R_3$, où Y représente un atome d'oxygène ou de soufre, n représente les chiffres 0 ou 1, m représente les chiffres 1, 2 ou 3, et $R^3$ représente un atome d'hydrogène ou un radical alkyle ou acyle comportant chacun 1 à 4 atomes de carbone, avec la condition que, lorsque m représente 1 et Y un atome d'oxygène, $R^3$ représente un radical alkyle comportant 1 à 4 atomes de carbone.

2. La 11β-(4-diméthylaminophényl)-17α-(but-1-yn-3-ényl)-17β-hydroxy-4,9-œstradiène-3-one,
la 11β-(4-diméthylaminophényl)-17α-(méthoxyméthyl)-17β-hydroxy-4,9-œstradiène-3-one,
la 11β-(4-diméthylaminophényl)-17α-(2-méthoxyéthyl)-17β-hydroxy-4,9-œstradiène-3-one,
la 11β-(4-diméthylaminophényl)-17α-(éthoxyméthyl)-17β-hydroxy-4,9-œstradiène-3-one,
la 11β-(4-diméthylaminophényl)-17α-(4-hydroxybut-1-(Z)-ényl)-17β-hydroxy-4,9-œstradiène-3-one,
la 11β-(4-diméthylaminophényl)-17α-(2-hydroxyéthyl)-17β-hydroxy-4,9-œstradiène-3-one,
la 11β-(4-diméthylaminophényl)-17α-(éthylthiométhyl)-17β-hydroxy-4,9-œstradiène-3-one,
la 11β-(4-diméthylaminophényl)-17α-(méthoxyméthyl)-17β-hydroxy-4,9-œstradiène-3-one-anti-oxime,
la 11β-(4-diméthylaminophényl)-17α-(méthoxyméthyl)-17β-hydroxy-4,9-œstradiène-3-one-syn-oxime,
la 11β-(4-diméthylaminophényl)-17α-(but-1-yn-3-ényl)-17β-hydroxy-4,9-œstradiène-3-one-anti-oxime,
la 11β-(4-diméthylaminophényl)-17α-(but-1-yn-3-ényl)-17β-hydroxy-4,9-œstradiène-3-one-syn-oxime.

3. Procédé de préparation de composés répondant à la formule générale I

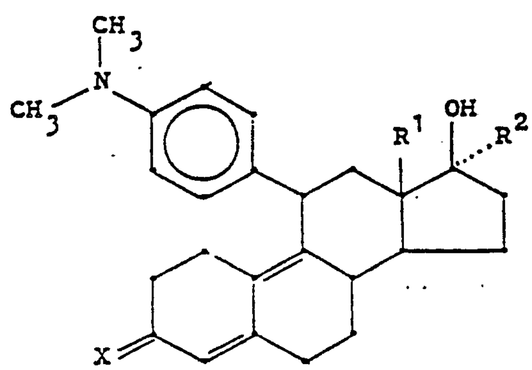

(I)

dans laquelle

X représente un atome d'oxygène ou un groupe hydroxyimino N~OH, où le groupe hydroxy peut être en position syn ou anti,

$R^1$ représente un groupe méthyle ou éthyle,

$R^2$ représente un groupe $—C{\equiv}C—CH{=}CH_2$, $—CH_2—(CH_2)_n—Y—R^3$ ou $—CH{=}CH—(CH_2)_m—Y—R^3$, où Y représente un atome d'oxygène ou de soufre, n représente les chiffres 0 ou 1, m représente les chiffres 1, 2 ou 3, et $R^3$ représente un atome d'hydrogène ou un radical alkyle ou acyle comportant chacun 1 à 4 atomes de carbone, avec la condition que, lorsque m représente 1 et Y un atome d'oxygène, $R^3$ représente un radical alkyle comportant 1 à 4 atomes de carbone,
caractérisé en ce qu'on soumet un composé de la formule générale II

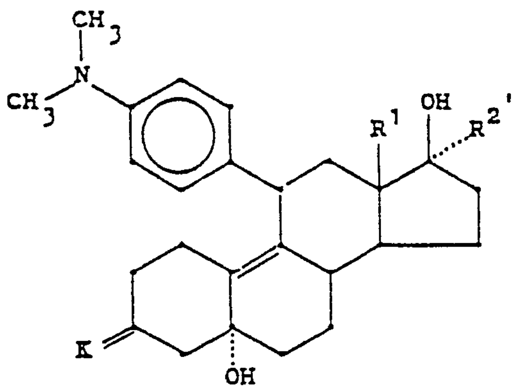

(II)

dans laquelle K représente un groupe cétonique bloqué sous la forme du cétal, du thiocétal, de l'oxime ou de la méthyloxime, $R^1$ a la même signification que celle donnée précédemment et $R^{2'}$ a la même signification que $R^2$, des groupes hydroxy ou mercapto éventuellement présents étant protégés, à l'action d'un agent de déshydratation qui est également capable de libérer la fonction protégée, en vue de l'élimination d'eau avec formation simultanée de la double liaison 4(5) et en ce qu'éventuellement on éthérifie ou estérifie un groupe hydroxy ou mercapto présent dans $R^2$, avec formation du produit de la formule générale I où X représente un atome d'oxygène, et en ce qu'éventuellement ensuite on fait réagir avec du chlorhydrate d'hydroxylamine en présence d'amines tertiaires, à des températures comprises entre — 20 et + 40 °C.

4. Compositions pharmaceutiques, caractérisées par une teneur en composés suivant l'une des revendications 1 et 2.

5. Utilisation de composés suivant l'une des revendications 1 et 2, pour la préparation de compositions pharmaceutiques.

**Revendication** (pour l'Etat contractant AT)

1. Procédé de préparation de composés répondant à la formule générale I

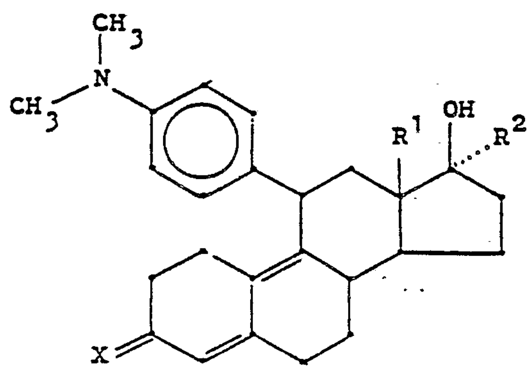

(I)

dans laquelle

X représente un atome d'oxygène ou un groupe hydroxyimino N~OH, où le groupe hydroxy peut être en position syn ou anti,

$R^1$ représente un groupe méthyle ou éthyle,

$R^2$ représente un groupe $—C{\equiv}C—CH{=}CH_2$, $—CH_2—(CH_2)_n—Y—R^3$ ou $—CH{=}CH—(CH_2)_m—Y—R^3$, où Y représente un atome d'oxygène ou de soufre, n représente les chiffres 0 ou 1, m représente les chiffres 1, 2 ou 3, et $R^3$ représente un atome d'hydrogène ou un radical alkyle ou acyle comportant chacun 1 à 4 atomes de carbone, avec la condition que, lorsque m représente 1 et Y un atome d'oxygène, $R^3$ représente un radical alkyle comportant 1 à 4 atomes de carbone,

caractérisé en ce qu'on soumet un composé de la formule générale II

(Voir Formule II page 19)

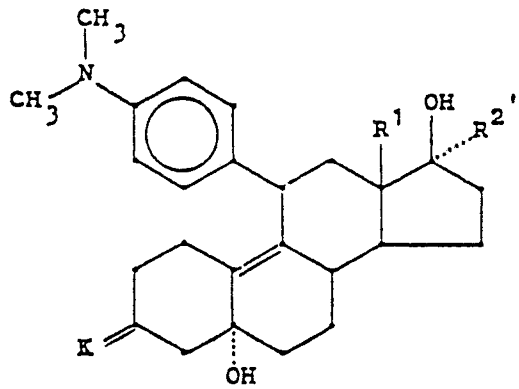

(II)

dans laquelle K représente un groupe cétonique bloqué sous la forme du cétal, du thiocétal, de l'oxime ou de la méthyloxime, $R^1$ a la même signification que celle donnée précédemment et $R^{2'}$ a la même signification que $R^2$, des groupes hydroxy ou mercapto éventuellement présents étant protégés, à l'action d'un agent de déshydratation qui est également capable de libérer la fonction protégée, en vue de l'élimination d'eau avec formation simultanée de la double liaison 4(5) et en ce qu'éventuellement on éthérifie ou estérifie un groupe hydroxy ou mercapto présent dans $R^2$, avec formation du produit de la formule générale I ou X représente un atome d'oxygène, et en ce qu'éventuellement ensuite on fait réagir avec du chlorhydrate d'hydroxylamine en présence d'amines tertiaires, à des températures comprises entre — 20 et + 40 °C.